Europäisches Patentamt

European Patent Office

Office européen des brevets

(19)

(11) Publication number: **0 018 419**

**B1**

(12) **EUROPEAN PATENT SPECIFICATION**

(45) Date of publication of patent specification: **18.04.84**

(21) Application number: **79900783.6**

(22) Date of filing: **13.07.79**

(86) International application number:
**PCT/JP79/00187**

(87) International publication number:
**WO 80/00274 21.02.80 Gazette 80/4**

(51) Int. Cl.³: **G 01 N 27/06,
G 01 N 33/48**

(54) **MILK INSPECTION APPARATUS.**

(30) Priority: **14.07.78 JP 85951/78**

(43) Date of publication of application:
**12.11.80 Bulletin 80/23**

(45) Publication of the grant of the patent:
**18.04.84 Bulletin 84/16**

(84) Designated Contracting States:
**CH DE FR GB**

(56) References cited:
**DE - A - 2 534 423
GB - A - 1 194 329
GB - A - 1 314 327
JP - A - 48 009 795
JP - A - 53 081 294
SU - A - 532 370
US - A - 2 898 549
US - A - 3 512 080
US - A - 3 989 009**

(73) Proprietor: **Eisai Co., Ltd.
6-10, Koishikawa 4-chome Bunkyo-ku
Tokyo 112 (JP)**

(72) Inventor: **TAKAHASHI, Toshio
2-31, Sakae 3-chome
Honjo-shi, Saitama 367 (JP)**

(74) Representative: **Sajda, Wolf E., Dipl.-Phys.
Patentanwälte Popp, Sajda, von Bülow, Hrabal &
Partner Postfach 86 06 24 Widenmayerstrasse 48
D-8000 München 86 (DE)**

Courier Press, Leamington Spa, England.

## Milk inspection apparatus

The present invention relates to an examination apparatus for milk drawn from the udders of a cow, comprising a trap equipped in each flow passage for the milk drawn from the corresponding udder, an electric conductivity measuring circuit connected to electrodes provided in each trap for measuring the electric conductivities of the milk flowing through each of the traps, a minimum value selecting circuit connected to said electric conductivity measuring circuit for selecting the minimum electric conductivity among said electric conductivities, a group of comparator circuits and an indication member connected to the group of comparator circuits for indicating the grade of quality of the milk.

When a milk cow suffers from mastitis or milk fever, the deterioration of milk and the decrease of quantity of secreted milk inflict heavy loss on the milk producer. Therefore, the early discovery of mastitis is important. •

At present, the abnormal milk detection method being utilized most widely is the California Mastitis Test (CMT) or a modification of CMT.

The extent of a coagulation reaction caused when CMT reagent is added to the sample milk is judged by this method. This is to measure semiqualitatively the number of somatic cells (mainly leukocytes) included in the milk.

However, these test operations require experience, being defective in that the judgement is apt to be governed by the subjective element, the difference between the individuality, or the physiological condition of the cow; therefore, the correct judgement cannot be achieved easily.

It is well known that mastitis and milk fever are diseases of the udders, and in the milk drawn from the cow suffering from these diseases, the concentration of Na-ions and Cl-ions is abnormally high, increasing the electric conductivity of the milk.

According to the results of observation of values of ingredients analysed of milk from each udder, the ingredient of milk from each udder of the same udders of the healthy cow is the same showing the same electric conductivity. However, in case the cow is suffering from mastitis it is scarcely observed that all milk from four udders is tainted and the ingredients of milk from all the udders show abnormal values. The abnormality is restricted to a part of the udders. This is because the udders do not have the same sensitivity to various stimulations and a certain udder is more affected sensitively than another udder being apt to suffer from mastitis easily.

Accordingly, it is necessary to examine the milk drawn from each udder instead of the mixed milk from four udders and to measure the electric conductivity of the milk from each udder. The electric conductivity of the milk is varied not only by a disease such as mastitis but also by the difference of the individuality, the physiological condition, the season and the temperature. Therefore, by comparing the conductivity of respective milk drawn from the udders, the mastitis can be surely judged; at the same time, the extent of disease affecting each udder can be estimated.

Generally in a milking operation, milk streams secreted in each udder automatically drawn by a milking machine are mixed instantaneously into the mixed milk. Therefore, the sampling is necessary to be done before the milking machine is fitted to the cow. Further, the state of the quality of milk changing during the operation of milking, cannot be checked.

An examination apparatus for milk drawn from the udders of a cow as set forth above is known for example from US—A—3 989 009. This publication discloses a measurement device for milk drawn from the udders and comprises a tube equipped in each flow passage to the milk drawn from the udder, an electric conductivity measuring circuit for measuring the electric conductivity of each milk passage, a minimum value selecting circuit connected to the electric conductivity measuring circuit for selecting the minimum conductivity and a group of gates which provide a minimum output signal.

In the examination apparatus according to US—A—3 989 009 the minimum conductivity of one fluid of a group of fluids is determined by comparing electrical conductivities. To this end, a minimum signal detector is connected to the output of sample and hold circuits and operates to determine the signal having the least amplitude. The output of the minimum signal detector is applied to a gate circuit which receives also the output of the sample and hold circuits. Accordingly, an enable output signal of the minimum signal detector enables only one of the analog conductivity signals applied to the gate circuit to its output, namely a minimum amplitude signal which is applied to one input of a divider. To the other input of this divider is applied a signal which represents the conductivities of each of the fluid streams which traverse fluid tubes. This divider calculates the ratio of the two input values.

It is clear that difficulties will arise when rather similar input signals are used in the device according to US—A—3 989 009 and applied to a divider disclosed therein. If a divider calculates the ratio of such values the result will be unity so that small differences of the values will hardly be recognized. There is no hint, however, in the US—A—3 989 009 to use a device incorporating a subtracting circuit instead of the divider and making use of additional comparator circuits with given limit values.

From DE—A—2 534 423 an apparatus is known for measuring impurity differences of a fluid especially milk supplied from different sources, for example the udders of a cow. Six comparator circuits are provided which are connected with electrodes of sensors arranged in each milk flow and which compare the milk resistivity differences between each other and a different udder. A comparator circuit compares the output signals of each udder with a reference value and a lamp lights up when the general signal level of the signals supplied from the four sensors exceeds a reference level provided from a potentiometer circuit.

So, the examination apparatus known from DE—A—2 534 423 provides an output signal when the comparison between any two sensors results in difference signals, and an indication is also provided when the level of any sensor signal is different in a certain amount from a reference level.

The operation of the apparatus according to DE—A—2 534 432 is more complicated in that the comparator compares the output signals of each of all the udder sensors with a reference value.

The object underlying the invention as claimed is to provide an examination apparatus for milk drawn from the udders of a cow which is capable of eliminating the drawbacks of the prior art. It solves the problem of how to design such an examination apparatus which is easy and reliable in operation, provides an inexpensive circuit arrangement and precise examination results within a short time period.

The invention is characterized in that a subtracting circuit is connected to the electric conductivity measuring circuit and the minimum value selecting circuit, the subtracting circuit having outputs which provide the differences in value between the minimum electric conductivity and each of said electric conductivities, the outputs of the subtracting circuit being connected to the group of comparator circuits, in that the group of comparator circuits, for comparing each of the outputs of the subtracting circuit with first and second reference values comprises first comparator circuits to which an upper limit value is inputted corresponding to the first reference value and second comparator circuits to which a lower limit is inputted corresponding to the second reference value, in that the indication member provides for the milk flowing through each of the traps indications of normal, abnormal and quasi-abnormal milk according to whether the corresponding output of the subtracting circuit is below the lower limit value, higher than the upper limit value, and intermediate between the upper and the lower limit value, respectively, and in that an additional comparator circuit is connected to the minimum value selecting circuit, the additional comparator circuit outputting a signal when the minimum electric conductivity value exceeds a third reference value, the signal being directly fed to the indication member for providing an indication that the whole milk drawn from the udders is abnormal.

With the examination apparatus according to the invention it is possible to check milk drawn from the udders of a cow automatically and continuously during the continuous milking operation. In so doing an early detection of abnormal milk streams is provided even when only one udder is affected, but of course also when two or more udders are affected. Moreover, it is possible to detect exactly abnormal milk streams when all the udders of a cow are affected since a corresponding output signal is delivered by the additional comparator circuit.

In the examination apparatus according to the invention the additional comparator circuit compares only the minimum electric conductivity value with a predetermined value so that only one comparison is necessary in order to detect any abnormality of the milk streams from all four udders. In this way the processing time of the examination apparatus is considerably shortened and the costs of the circuit arrangement are reduced.

In each trap of the examination apparatus, a certain quantity of milk drawn from each other is always stored in such a way that the stored milk is exchanged successively with milk newly sucked out through the corresponding teatcups.

Although the milk from each udder is drawn out intermittently by the intermittend vacuum sucking of the milk machine, the measuring electrodes are always dipped into the milk from the udders under examination and are protected against disturbances due to intermission or bubble of the milk from the udders.

Each of the four traps is provided with a pair of measuring electrodes of metal such as platinum, stainless steel and the like. These electrodes are electrically insulated from each other and produce output signals corresponding to the electric conductivity of the milk from the udder when an AC input signal with a constant amplitude is applied.

The measuring circuit receives output signals from four sets of measuring electrodes, generating signals for the value of the electric conductivity of each udder. A minimum value selection circuit provides for a reference value and other measured values are compared with the reference value for obtaining respective value differences. The indication lamps indicate for each udder to which grade of quality the milk drawn therefrom belongs wherein the grades of quality are predetermined by the value differences with respect to the reference value. If the value of difference exceeds a predetermined level, a warning is given by an alarm buzzer. Thus, the milker is alarmed by the buzzer whether or not abnormality exists, and he can recognize by observing the indication lamp which udder is abnormal. Therefore, the milker can detect the abnormal milk stream and take early counter-measures for treating the cow suffering from a disease, thus obtaining an extremely economical effect.

Preferred ways of carrying out the invention are described below with reference to drawings, wherein:

Fig. 1 is a schematic view of a preferred embodiment of the examination apparatus for milk from the quarter mamma according to the present invention,

Fig. 2 is a block diagram of the examination apparatus shown in Fig. 1, and

Fig. 3 is a concrete circuit diagram of a grouping circuit of Fig. 2.

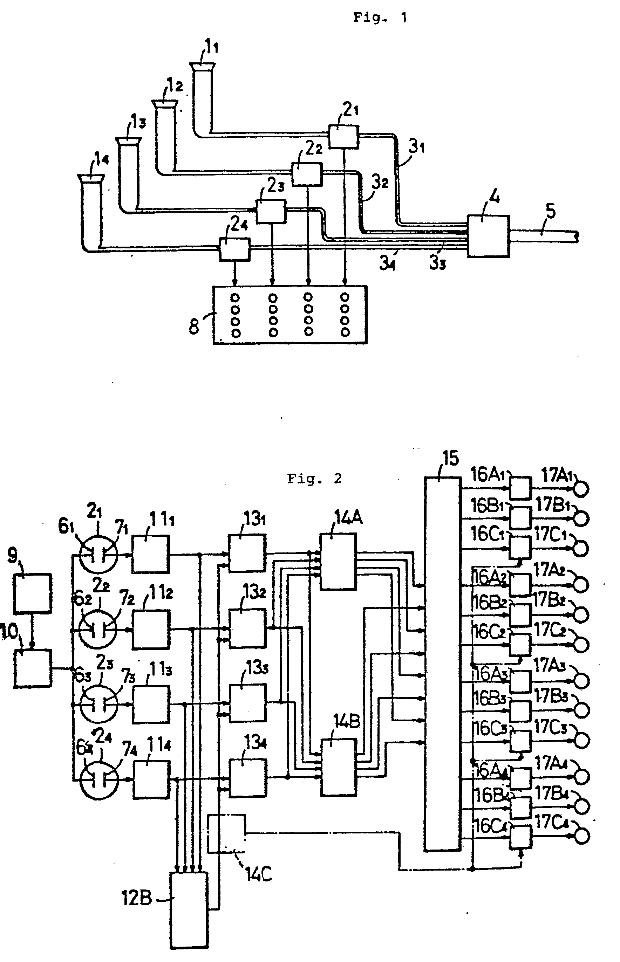

In Fig. 1, references $1_1$, $1_2$, $1_3$ and $1_4$ are teatcups to be fitted to the udders of a milk cow. These teatcups $1_1$, $1_2$, $1_3$ and $1_4$ are connected to a milkclaw 4 through flow ways (conduits) $3_1$, $3_2$, $3_3$ and $3_4$ with respective traps $2_1$, $2_2$, $2_3$ and $2_4$. In the milkclaw 4, the milk streams from the udders are joined together to be sent to a transmitting pipe 5. Each trap is provided with a pair of metallic electrodes $6_1$ and $7_1$, $6_2$ and $7_2$, $6_3$ and $7_3$, and $6_4$ and $7_4$ which are harmless to man from a hygienical point of view. These electrodes are connected to the examination apparatus 8. According to Fig. 2 showing the details of the examination apparatus, a sinusoidal wave oscillation circuit 9 is connected to each of the electrodes $6_1$, $6_2$, $6_3$ and $6_4$ on one side of the traps through a buffer amplifier 10.

Each electrode on the opposite side $7_1$, $7_2$, $7_3$ and $7_4$ is connected to a respective electric conductivity measuring circuit $11_1$, $11_2$, $11_3$ and $11_4$. These measuring circuits are connected to a minimum value selection circuit 12B and subtraction circuits $13_1$, $13_2$, $13_3$ and $13_4$. These subtraction circuits $13_1$, $13_2$, $13_3$ and $13_4$ are connected through comparator circuits 14A and 14B and a grouping circuit 15 to lamps and buzzer driving circuits $16A_1$, $16B_1$ and $16C_1$, $16A_2$, $16B_2$, and $16C_2$, $16A_3$ and $16B_3$ and $16C_3$, and $16A_4$, $16B_4$ and $16C_4$, and also to indication lamps $17A_1$, $17B_1$ and $17C_1$, $17A_2$, $17B_2$ and $17C_2$, $17A_3$, $17B_3$ and $17C_3$, and $17A_4$, $17B_4$ and $17C_4$.

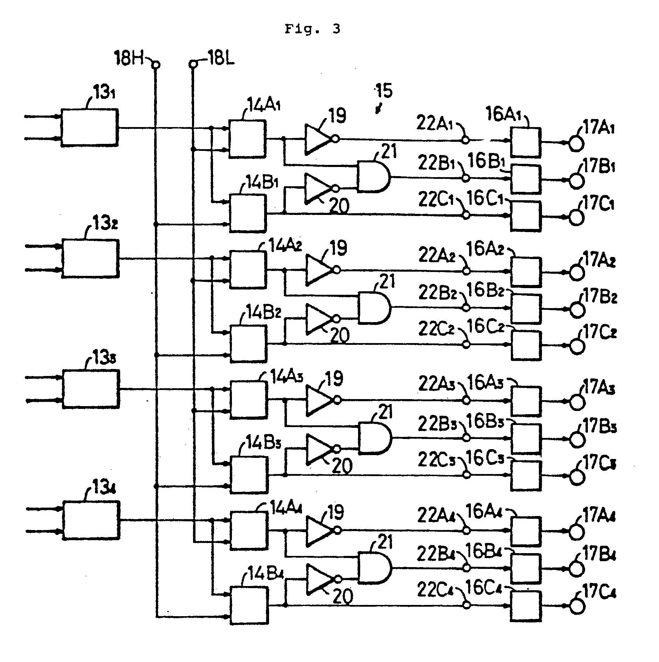

The comparator circuits and lamp and buzzer driving circuits $16A_1$, $16B_1$, $16C_1$, ..., $16A_4$, $16B_4$, $16C_4$ are constructed as shown in Fig. 3. That is, a low level reference value input terminal 18L is connected to one of the group of comparator circuits $14A_1$, $14A_2$, $14A_3$ and $14A_4$, while a high level reference value input terminal 18H is connected to the other group of comparator circuits $14B_1$, $14B_2$, $14B_3$ and $14B_4$. The grouping circuit 15 is composed of two NOT circuits 19 and 20 and one AND circuit 21. At the first output terminals $22A_1$, $22A_2$, $22A_3$ and $22A_4$, signals of the normal milk are provided, and at the second output terminals $22B_1$, $22B_2$, $22B_3$ and $22B_4$ signals of the quasi-abnormal milk (when it is assumed that the milk may be abnormal) are delivered, while at the

third output terminals $22C_1$, $22C_2$, $22C_3$ and $22C_4$, signals of abnormal milk are to be furnished. These three groups of output terminals $22A_1$, $22A_2$, $22A_3$ and $22A_4$, $22B_1$, $22B_2$, $22B_3$ and $22B_4$ and $22C_1$, $22C_2$, $22C_3$ and $22C_4$ are connected to normal milk indicator lamps $17A_1$, $17A_2$, $17A_3$ and $17A_4$, quasi-abnormal milk indicator lamps $17B_1$, $17B_2$, $17B_3$ and $17B_4$, and abnormal milk indicator lamps $17C_1$, $17C_2$, $17C_3$ and $17C_4$ through the driving circuits $16A_1$ ..., $16B_1$ ..., $16C_1$ ..., $16C_4$. Although the buzzer is not shown, it sounds when an output appears at any one of the second or third terminals $22B_1$ ... $22B_4$, and $22C_1$ ... $24C_4$.

The operation of the apparatus according to the invention will be described hereinafter.

As the reference difference in value between udders applied to the lower level input terminal 18L and the higher level input terminal 18H, $5 \times 10^{-4}$ S/cm and $15 \times 10^{-4}$ S/cm are taken, respectively, where S/cm (S=Siemens) is the conductivity. Accordingly, the milk with a measured value lower than $5 \times 10^{-4}$ S/cm is judged to be normal, while that having a value higher than $15 \times 10^{-4}$ S/cm is judged to be abnormal, and further the milk having a value between these two values is judged to be quasi-abnormal.

In the state established as described above, when the milk is vaccum-sucked intermittently by the milking machine from the side of milkclaw 4, the milk streams from teatcups $1_1$, $1_2$, $1_3$ and $1_4$ passing through flow ways $3_1$, $3_2$, $3_3$ and $3_4$ are stored for a time in each one of traps $2_1$, $2_2$, $2_3$ and $2_4$ and thereafter joined in the milkclaw 4.

The sinusoidal output signal from the oscillation circuit 9 is applied to each one of electrodes $6_1$, $6_2$, $6_3$ and $6_4$ on one side through the buffer amplifier 10, and an alternating current flows to each one of electrodes $7_1$, $7_2$, $7_3$ and $7_4$ on the opposite side, the current corresponding to the electric conductivity of the milk from each udder in traps $2_1$, $2_2$, $2_3$ and $2_4$, respectively. In each conductivity measuring circuit $11_1$, $11_2$, $11_3$ and $11_4$, a DC signal showing the electric conductivity of milk from an udder is taken out as an output. These four signals showing the electric conductivity are transmitted to a minimum value selection circuit 12B, and the minimum conductivity signal of these signals is selected. In subtraction circuits $13_1$, $13_2$, $13_3$ and $13_4$, the subtraction is performed between the minimum conductivity and the other conductivity. Each circuit delivers a signal of differential conductivity. The differential conductivity is compared in one group of comparator circuits $14A_1$, $14A_2$, $14A_3$ and $14A_4$ with the reference value of the lower level, that is $5 \times 10^{-4}$ S/cm. When the former is higher than the latter, an output appears. In the other group of comparator circuits $14B_1$, $14B_2$, $14B_3$ and $14B_4$, the comparison is performed with the higher level reference value of $15 \times 10^{-4}$ S/cm. If

the differential is lower than this value, an output appears. The output signals from the groups of comparators $14A_1$, $14A_2$, $14A_3$ and $14A_4$, $14B_1$, $14B_2$, $14B_3$ and $14B_4$ are transmitted to the grouping circuit 15 in which signals are divided into three groups. In other words, when there is no output from one group of comparator circuits $14A_1$, $14A_2$, $14A_3$ and $14A_4$, outputs appear only at the first output terminals $22A_1$, $22A_2$, $22A_3$ and $22A_4$ through NOT circuits 19, 19, 19 and 19, thereby operating driving circuits $16A_1$, $16A_2$, $16A_3$ and $16A_4$ and lighting normal milk indication lamps $17A_1$, $17A_2$, $17A_3$ and $17A_4$. Next, when there appear outputs only from one group of comparator circuits $14B_1$, $14B_2$, $14B_3$ and $14B_4$ and there is no output from the other group of comparator circuits $14B_1$, $14B_2$, $14B_3$ and $14B_4$, signals from one group of the comparator circuits $14A_1$, $14A_2$, $14A_3$ and $14A_4$ are applied to each input terminal on the one side, and signals inverted by NOT circuits 20 are applied to each terminal on the opposite side of the AND circuit. Therefore, outputs come out from the second terminals $22B_1$, $22B_2$, $22B_3$ and $22B_4$, thereby activating the driving circuits $16B_1$, $16B_2$, $16B_3$ and $16B_4$ and lighting the quasi-abnormal milk indication lamps $17B_1$, $17B_2$, $17B_3$ and $17B_4$ and at the same time sounding the buzzer. Further, when there are outputs from both groups of comparator circuits $14A_1$, $14A_2$, $14A_3$ and $14A_4$ and $14B_1$, $14B_2$, $14B_3$ and $14B_4$, the outputs appear only at the third output terminals $22C_1$, $22C_2$, $22C_3$ and $22C_4$, thereby actuating the driving circuits $16C_1$, $16C_2$, $16C_3$ and $16C_4$, lighting the abnormal milk indication lamps $17C_1$, $17C_2$, $17C_3$ and $17C_4$ and sounding the buzzer.

Thus, which milk stream is normal, quasi-normal or abnormal is individually indicated, and the extent of quality of milk can be judged.

In the above embodiment, the examination apparatus is so constructed as to judge the milk from the udders individually. Namely, when a milk cow suffers from a disease such as mastitis or heat fever, normally all of the udders are affected at the same time, but in most cases only one or two of the udders are affected. Therefore, if any affected udder is detected, the milking operation can be stopped and the proper treatment can be taken.

The judgement of whether the milk from the udders is normal or not (abnormal), is important and advantageous in livestock farming.

In Fig. 2, among the outputs from the conductivity measuring circuits $11_1$, $11_2$, $11_3$ and $11_4$, the minimum conductivity value obtained in the minimum selection circuit 12B is to be transmitted to the subtraction circuits $13_1$, $13_2$, $13_3$ and $13_4$ as it is used for obtaining the differential value. In many cases, it is observed that the abnormality is found in the limited portion of the four udders, as described above.

It is in rare cases only that all four udders are affected so that the milk streams from these udders each show high conductivity values so as to give almost no difference to the differential value between the udders.

Furthermore, as shown in Fig. 2 by chain lines, a comparator circuit 14C in which the abnormal milk judging reference value is already set is provided and connected to the minimum value selection circuit 12B as shown in the drawing so that when the minimum conductivity value obtained by the minimum selection circuit 12B exceeds the reference value of this comparator circuit 14C, all of the milk streams from the udders are considered to be abnormal and to show abnormally high conductivity, and the comparator circuit 14C is connected to the lamp and buzzer driving circuits $16C_1$, $16C_2$, $16C_3$ and $16C_4$, directly activating the abnormal milk indicator lamps $17C_1$, $17C_2$, $17C_3$ and $17C_4$. When the value of the minimum value selection circuit 12B does not exceed the reference value of the comparator circuit 14C, the signal from the selection circuit 12B is transmitted to subtraction circuits $13_1$, $13_2$, $13_3$ and $13_4$. If constructed as described above, even in the case where the high conductivity value is shown, but there is no difference between the differential values of the milk streams from the udders these milk streams can be detected to be abnormal without exception, and thus the examination can be made more complete.

As the present invention is constituted as described heretofore, milk drawn from the udders can be examined continuously in the course of the milking operation. Consequently, the state of variation of the quality of milk can be verified by a continuous examination throughout the time of the milking operation.

Further, as the electrodes are equipped in the traps, even if the milk from the udders flows intermittently or bubbles are formed in the flow way, the apparatus will not operate erroneously.

As described above, the examination apparatus according to the present invention is attached to the milking machine for automatically drawing milk from the cow. This apparatus can be attached to a machine of the claw type of course, and also to that of the suspension type. Further, the apparatus can be attached to machines of any size, such as small ones which draw milk from a milk cow at a time and large ones which treat several cows at a time.

**Claim**

1. An examination apparatus for milk drawn from the udders of a cow, comprising a trap $(2_1$—$2_4)$ equipped in flow passage $(3_1$—$3_4)$ for the milk drawn from the corresponding udder, an electric conductivity measuring circuit $(11_1$—$11_4)$ connected to electrodes $(6_1$—$6_4$; $7_1$—$7_4)$ provided in each trap $(2_1$—$2_4)$ for measuring the electric conductivities of the milk flowing through each of the traps, a minimum value selecting circuit (12B) connected to said electric conductivity measuring circuit (11$_1$—11$_4$) for selecting the minimum electric conductivity among said electric conductivities, a group of comparator circuits (14A$_1$—14A$_4$; 14B$_1$—14B$_4$) and an indication member (17A—17C) connected to the group of comparator circuits for indicating the grade of quality of the milk, characterized in that a subtracting circuit (13$_1$—13$_4$) is connected to said electric conductivity measuring circuit (11$_1$—11$_4$) and the minimum value selecting circuit (12B), the subtracting circuit having outputs which provide the differences in value between the minimum electric conductivity and each of said electric conductivities, said outputs of the subtracting circuits being connected to the group of comparator circuits, in that said group of comparator circuits (14A$_1$—14A$_4$; 14B$_1$—14B$_4$) for comparing each of said outputs of the subtracting circuit with first and second reference values comprises first comparator circuits (14B$_1$—14B$_4$) to which an upper limit value is inputted corresponding to said first reference value and second comparator circuits (14A$_1$—14A$_4$) to which a lower limit is inputted corresponding to said second reference value, in that said indication member (17A—17C) provides for the milk flowing through each of the traps indications of normal, abnormal and quasi-abnormal milk according to whether the corresponding output of the subtracting circuit (13$_1$—13$_4$) is below the lower limit value, higher than the upper limit value, and intermediate between the upper and the lower limit value, respectively, and in that an additional comparator circuit (14C) is connected to the minimum value selecting circuit, said additional comparator circuit outputting a signal when the minimum electric conductivity value exceeds a third reference value, said signal being directly fed to the indication member for providing an indication that the whole milk drawn from the udders is abnormal.

**Patentanspruch**

1. Vorrichtung zur Kontrolle von Milch, die von dem Euter einer Kuh abgezogen wird, mit einem Auffänger (2$_1$—2$_4$), der in jedem Strömungsweg (3$_1$—3$_4$) für die von der jeweiligen Zitze abgezogenen Milch angeordnet ist, mit einer Meßschaltung (11$_1$—11$_4$) für die elektrische Leitfähigkeit, die zur Messung der elektrischen Leitfähigkeiten der durch die jeweiligen Auffänger fließenden Milch an Elektroden (6$_1$—6$_4$; 7$_1$—7$_4$) angeschlossen ist, die in dem jeweiligen Auffänger (2$_1$—2$_4$) vorgesehen sind, mit einer Minimalwert-Wählschaltung (12B), die an die Meßschaltung (11$_1$—11$_4$) für die elektrische Leitfähigkeit angeschlossen ist, um die minimale elektrische Leitfähigkeit unter den elektrischen Leitfähigkeiten zu wählen, mit einer Gruppe von Komparator-

schaltungen (14A$_1$—14A$_4$; 14B$_1$—14B$_4$), und mit einem Anzeigeelement (17A—17C), das an die Gruppe von Komparatorschaltungen angeschlossen ist, um den Qualitätsgrad der Milch anzuzeigen, dadurch gekennzeichnet, daß eine Subtraktionsschaltung (13$_1$—13$_4$) an die Meßschaltung (11$_1$—11$_4$) für die elektrische Leitfähigkeit und an die Minimalwert-Wählschaltung (12B) angeschlossen ist, wobei die Subtraktionsschaltung Ausgänge besitzt, welche die Differenzwerte zwischen der minimalen elektrischen Leitfähigkeit und jeder der elektrischen Leitfähigkeiten liefern und welche an die Gruppe von Komparatorschaltungen angeschlossen sind, daß die Gruppe von Komparatorschaltungen (14A$_1$—14A$_4$; 14B$_1$—14B$_4$) zum Vergleich der jeweiligen Ausgangssignale der Subtraktionsschaltung mit ersten und zweiten Referenzwerten eine erste Komparatorschaltung (14B$_1$—14B$_4$), in die ein dem ersten Referenzwert entsprechender oberer Grenzwert eingegeben wird, und eine zweite Komparatorschaltung (14A$_1$—14A$_4$) aufweist, in die ein dem zweiten Referenzwert entsprechender unterer Grenzwert eingegeben wird, daß das Anzeigeelement (17A—17C) für die durch die jeweiligen Auffänger fließende Milch Anzeigen für normale, anormale und quasi-anormale Milch in Abhängigkeit davon liefert, ob das entsprechende Ausgangssignal der Subtraktionsschaltung (13$_1$—13$_4$) niedriger als der untere Grenzwert, höher als der obere Grenzwert bzw. zwischen dem oberen und dem unteren Grenzwert liegt, und daß eine zusätzliche Komparatorschaltung (14C) an die Minimalwert-Wählschaltung angeschlossen ist, wobei die zusätzliche Komparatorschaltung ein Signal abgibt, wenn der Minimalwert der elektrischen Leitfähigkeit einen dritten Referenzwert überschreitet, und wobei dieses Signal direkt dem Anzeigeelement zugeführt wird, um eine Anzeige zu liefern, daß die gesamte vom Euter abgezogene Milch anormal ist.

**Revendication**

1. Appareil de contrôle du lait tiré des tétines d'une vache, comprenant une trappe (2$_1$—2$_4$) disposée dans chaque passage d'écoulement (3$_1$—3$_4$) pour le lait tiré de la tétine correspondante, un circuit de mesure de la conductivité électrique (11$_1$—11$_4$) relié à des électrodes (6$_1$—6$_4$; 7$_1$—7$_4$) prévus dans chaque trappe (2$_1$—2$_4$) pour mesurer les conductivités électriques du lait s'écoulant à travers chacune des trappes, un circuit de sélection de la valeur minimum (12B) relié audit circuit de mesure de la conductivité électrique (11$_1$—11$_4$) pour sélectionner la conductivité électrique minimale parmi lesdites conductivités électriques, un groupe de circuits comparateurs (14A$_1$—14A$_4$; 14B$_1$—14B$_4$) et un organe indicateur (17A—17C) relié au groupe de circuits comparateurs pour indiquer le niveau de qualité du lait, caractérisé en ce qu'un circuit sous-

tractif ($13_1$—$13_4$) est relié audit circuit de mesure de la conductivité électrique ($11_1$—$11_4$) et au circuit de sélection de la valeur minimale (12B), ce circuit soustractif présentant des sorties qui fournissent les différences de valeur entre la conductivité électrique minimale et chacune desdites conductivités électriques, lesdites sorties du circuit soustractif étant reliées au groupe des circuits comparateurs, en ce que ledit groupe de circuits comparateurs ($14A_1$— $14A_4$; $14B_1$—$14B_4$) pour comparer chacune desdites sorties du circuit soustractif avec des premières et secondes valeurs de référence comportent des premier circuits comparateurs ($14B_1$—$14B_4$) auxquels une valeur limite supérieure est injectée en correspondance à ladite première valeur de référence et des seconds circuits comparateurs ($14A_1$—$14A_4$) auxquels une valeur limite inférieure est injectée en correspondance à ladite seconde valeur de référence, en ce que ledit organe indicateur (17A—17C) délivre pour le lait s'écoulant à travers chacune des trappes des indications d'un lait normal, anormal et quasi normal selon que la sortie correspondante du circuit soustractif ($13_1$—$13_4$) est en-dessous de la valeur limite inférieure, supérieur à la valeur limite supérieure et intermédiaire entre la valeur limite supérieure et inférieure, respectivement, et en ce qu'un circuit comparateur additionnel (14C) est relié au circuit de sélection de la valeur minimale, ledit circuit comparateur additionnel émettant un signal lorsque la valeur de la conductivité électrique minimale dépasse une troisième valeur de référence, ledit signal étant directement fourni à l'organe indicateur pour indiquer que la totalité du lait tiré des tétines est anormal.

Fig. 1

Fig. 2

Fig. 3